# EUROPEAN PATENT SPECIFICATION

(11) **EP 0 337 774 B1**
(45) Date of publication and mention of the grant of the patent: **31.05.1995**
(21) Application number: 89303644.2
(22) Date of filing: 12.04.1989
(51) Int. Cl.: C07K 5/06, C07D 209/00, A61K 38/00, A61K 31/00

(54) **CCK antagonists**
CCK-Antagonisten
Antagonistes de CCK

(30) Priority: 12.04.1988 US 180310; 12.08.1988 US 231493
(43) Date of publication of application: 18.10.1989
(73) Proprietor: BIOMEASURE, INC., Hopkinton Massachusetts 01748 (US)
(72) Inventor: Kim, Sun Hyuk, Chestnut Hill, MA 02167 (US)
(74) Representative: Deans, Michael John Percy

(56) References cited:
- EP-A- 0 106 281
- EP-A- 0 224 151
- EP-A- 0 230 151

## Description

This invention relates to cholecystokinin (CCK) antagonists.

Chang et al., 230 Science 177 (1985), describes CCK as "a hormonal regulator of pancreatic and gastric secretion, contraction of the gallbladder, and gut motility," and states that "CCK also exists in the brain and may have an equally important role as a central nervous system transmitter." Chang et al. further mentions the CCK antagonists have "potential therapeutic utilities" and describes the compound asperlicin, which has the structure 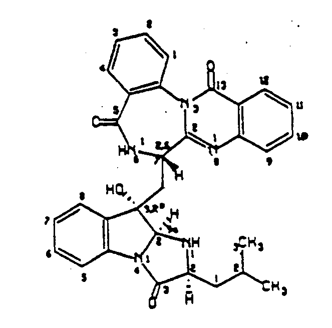
, as a CCK antagonist.

Rovati et al., U.S. Pat. 4,000,297, discloses compounds of the structure 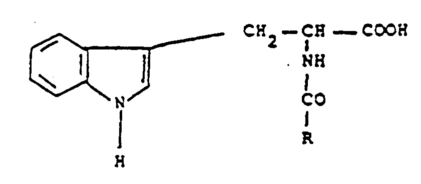
in which R includes mono and poly substituted phenyl groups, and R¹ includes hydroxyl, an aniline group substituted at the para position with a carboxylic acid or ester thereof, an amino group substituted with phenylacetic acid or ester derivative thereof, or an alkoxy group terminating with an amino group. The compounds are described as having an antispastic effect on the smoother muscle of the gastroenteric tract, as regulating gastric secretion, and as being protective of gastroenteric mucosa.

EP-A-0230151 describes compounds having the formula: 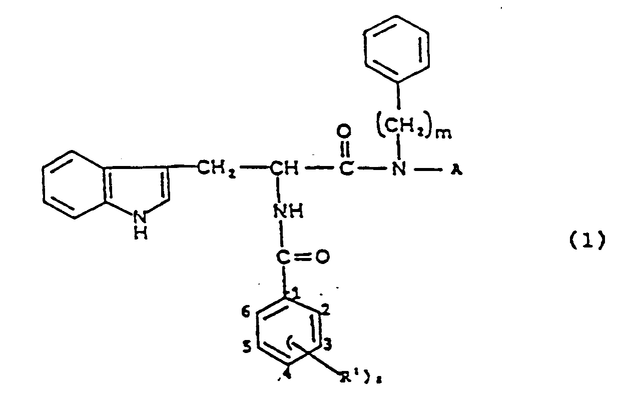
or a pharmaceutically acceptable salt thereof, wherein each R¹ is, independently, an alkyl group having 1 to 5, inclusive, carbon atoms, an alkoxy group having 1 to 5, inclusive, carbon atoms, a halogen, amino, hydroxy, nitro, cyano, carboxyl, trifluoromethyl, ethyl carboxylate, or a hydrogen; m is an integer between 0 and 2, inclusive; and A is either 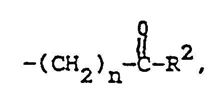
where n is an integer between 1 and 5, inclusive, and R² is hydroxy, an alkoxy group having 1 to 5, inclusive, carbon atoms, aralkoxy (e.g., benzyloxy), aralkyl (e.g., benzyl), amino, an alkyl group having 1 to 5, inclusive, carbon atoms, an alkylamino group having 1 to 5, inclusive, carbon atoms, a dialkylamino group with each alkyl group, independently having 1 to 5, inclusive, carbon atoms, a cycloalkylamino group wherein the ring has 4 to 6, inclusive, carbon atoms (e.g., pyrrolidino, piperidino. N-methylpiperazino), or morpholino; or A is an alkyl group having 1 to 5, inclusive, carbon atoms, a hydroxyalkyl group having 1 to 5, inclusive, carbon atoms, an alkoxyalkyl group having 2 to 8, inclusive, carbon atoms, an aralkoxyalkyl group having 8 to 14, inclusive, carbon atoms, an aryl group (e.g., phenyl, toluyl) having 6 to 14, inclusive, carbon atoms, an aralkyl group (e.g., benzyl, phenylethyl) having 6 to 14, inclusive, carbon atoms, and a cycloalkyl group having 3 to 12, inclusive, carbon atoms, and the synthesis of such compounds.

In one aspect, the invention features compounds having the formula: 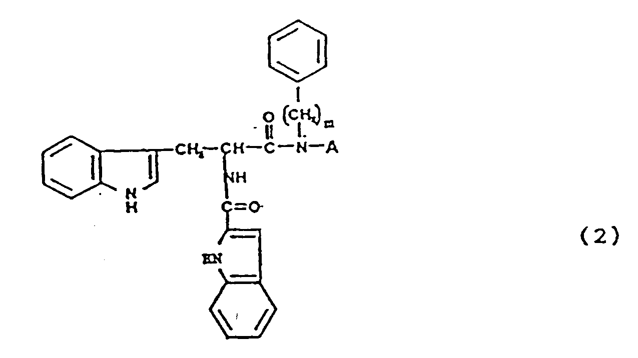
or a pharmaceutically acceptable salt thereof, wherein m is 0, 1 or 2, inclusive; and A is either 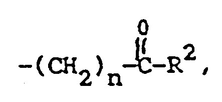
where n is an integer between 1 and 5, inclusive, and R² is hydroxy, an alkoxy group having 1 to 5, inclusive, carbon atoms, aralkoxy (e.g., benzyloxy), aralkyl (e.g., benzyl), amino, an alkyl group having 1 to 5, inclusive, carbon atoms, an alkylamino group having 1 to 5, inclusive, carbon atoms, a dialkylamino group with each alkyl group, independently, having 1 to 5, inclusive, carbon atoms, a cycloalkylamino group wherein the ring has 4 to 6, inclusive, carbon atoms (e.g.. pyrrolidino, piperidino, N-methylpiperazino), or morpholino; or A is an alkyl group having 1 to 5, inclusive, carbon atoms, a hydroxyalkyl group having 1 to 5, inclusive, carbon atoms, an alkoxyalkyl group having 2 to 8, inclusive, carbon atoms, an aralkoxyalkyl group having 8 to 14, inclusive, carbon atoms, an aryl group (e.g., phenyl, toluyl) having 6 to 14, inclusive, carbon atoms, an aralkyl group (e.g., benzyl, phenylethyl) having 6 to 14, inclusive, carbon atoms, or a cycloalkyl group having 3 to 12, inclusive, carbon atoms.

In preferred embodiments of the invention of formula (2), the tryptophan residue is of the L-configuration, m is 2, and R² is an alkoxy group or hydroxy. Preferred compounds include α-N-(2-indolylcarbonyl)-L-tryptyl-N'-phenylethylglycine and α-N-(2-indolylcarbonyl)-L-tryptyl- N'-phenyl- ethylglycineethylester; or a pharmaceutically acceptable salt thereof.

In other preferred embodiments, a therapeutically effective amount of the therapeutic compounds and a pharmaceutically acceptable carrier substance, e.g., magnesium carbonate or lactose, form a therapeutic composition, e.g., a pill, tablet, capsule, or liquid, for oral administration to a patient; a liquid or an ointment capable of being administered transdermally, nasally, rectally, or sublingualy; a liquid capable of being adminstered intravenously, parenterally, subcutaneously, or intraperitoneally; or an oral or a parenteral sustained release formulation.

The compounds of the invention are effective cholecystokinin antagonists and as such are effective in treating acute pancreatitis; in treating gastric, peptic, and duodenal ulcers; and in suppressing appetite. They are also effective, alone or in combination with other chemotherapeutic agents, in the treatment of pancreatic cancers; this activity is believed to occur because of antagonism to the action of cholecystokinin in inducing pancreatic hyperplasia in the presence of known carcinogens, e.g., nitrosamine. The compounds are stable, inexpensive to make, and non-toxic.

Other features and advantages of the invention will be apparent from the following description of the preferred embodiments thereof, and from the claims.

We now describe the structure, synthesis, and use of preferred embodiments of the invention.

The compounds of the invention have the general formulae (2) recited above. Examples of preferred compounds within these formulae are those referred to as preferred embodiments above.

The compounds of the invention are N-substituted (D- or L-) tryptyl N'-disubstituted glycine or N-substituted (D- or L-) tryptyl N'-disubstituted amide derivatives.

The compounds can also be provided in the form of pharmaceutically acceptable salts. Examples of suitable salts include those formed with hydrochloric, hydrobromic, sulfuric, maleic, acetic, or fumaric acid; potassium, sodium, or aluminum hydroxide; or dicyclohexylamine.

Compounds of formula (2) above can be synthesized as follows. First, a compound of formula (5) 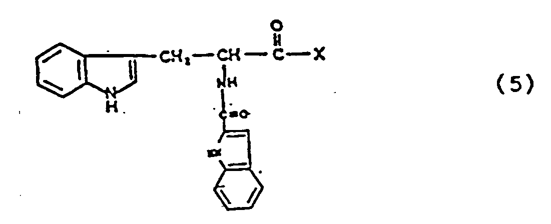
, where X represents a hydroxyl or carboxylic acid activating group, e.g., a halogen such as chlorine, is condensed with a secondary amino compound of formula (6) 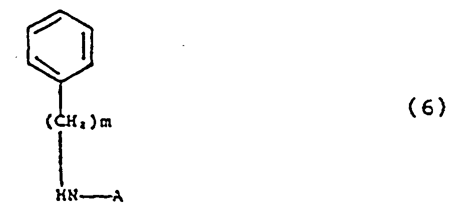
where m and A are defined as before.

The corresponding acids are then prepared from these esters by hydrolyzing the esters with aqueous base. Amides can be prepared by treating the corresponding esters with ammonia or an amine.

Compounds within formulae (5) and (6) are commercially available; alternatively they can be synthesized according to standard methods, e.g., as described in Greenstein, et al., 1961, supra; 1962, supra; 1949, supra; 1969, supra; and 1972, supra.

The condensation reactions are preferably carried out as described above for compounds synthesized from formulae (3) and (4). The intermediate and final products are isolated and purified by standard methods e.g., column chromatography or crystallization. Purity is determined using chromatographic, spectroscopic, and chemical analysis.

Specific compounds within forumula (2) are made as follows.

### α-N-(2-Indolylcarbonyl)-L-tryptyl-N′-phenylethylglycineethylester

The first step is the preparation of α-N-(2-indolylcarbonyl)-L-tryptophan, as follows. To an ice-cooled solution of L-tryptophan methylester HCl (1.0g), indole-2-carboxylic acid (0.63g), diethylcyanophosphonate (0.71g) in 10ml dimethylformide is added dropwise 1.65ml triethylamine, and the reaction mixture is stirred at 0.5°C for 1 hour and then at room temperature overnight. Volatile substances are removed in vacuo, and the residue is partitioned between chloroform and water. Precipitate (1.2g) is collected by filtration, washed with chloroform, and then dried. TLC: (silica gel; CHCl₃/MeOH = 9:1). Rf = 0.71.

To a suspension of α-N-(2-indolylcarbonyl)-L-tryptophan methylester (1g) in 20ml ethanol is added 3ml 1N NaOH, and after 1 hour stirring at room temperature, solvent is removed in vacuo to a small volume (∼5ml). The resulting solution is diluted with water and acidified with dilute HCl to pH 2-3. Pale pink solid (950mg) is collected by filtration; washed with water, and then dried. TLC: (silica gel; CHCl₃/MeOH/HOAC = 9:1:0.1). Rf = 0.32.

The acid is then condensed with the N′-phenylethyl substituted ethyl ester of glycine, as follows.

To an ice-cooled solution of α-N-(2-indolylcarbonyl)-L-tryptophan (0.8g), 1-hydroxybenzotriazole (0.65g) and N-phenylethylglycine ethylester (0.51g) in dimethylformide-dichloromethane (1:1, 20ml) is added a cold solution of dicyclohexylcarbodiimide (0.55g) im 1ml dichloromethane. The mixture is stirred at 0.5°C for 1 hour and then at room temperature overnight.

The mixture is then filtered, and solvents are evaporated in vacuo to dryness. The residue is dissolved in chloroform, washed with 5% aqueous NaHCO₃ and water, and dried over anhydrous MgSO₄. After evaporation of solvent, the residue is chromatographed on silica gel (45g) using chloroform-acetone (9:1) as eluant. Appropriate fractions are pooled and the solvent removed in vacuo to dryness. The pale yellow solid is recrystallized from ethanol to form a colorless solid. 0.44g TLC: (silica gel; CHCl₃/acetone = 9:1). Rf = 0.34.

### α-N-(2-Indolylcarbonyl)-L-tryptyl-N′-phenylethylglycine

To a suspension of α-N-(2-indolylcarbonyl)-L-tryptyl-N′-phenylethylglycine ethylester (200mg) in 2ml ethanol is added 1ml 2N NaOH. After 1 hour stirring at room temperature, most of the ethanol is removed in vacuo. The solution is then acidified with dilute HCl to pH 2-3. Colorless solid (130mg) is collected by filtration, washed with water, and then dried. TLC: (silica gel); CHCl₃/MeOH/HOAC = 9:1:0.1). Rf = 0.55.

When administered to a patient (e.g., orally, intravenously, parenterally, nasally, or by suppository), the compounds are effective in the treatment of gastric, peptic, and duodenal ulcers; in the treatment of acute pancreatitis; and in suppressing appetite.

The compounds can be administered to a human patient in a dosage of 0.1 - 50mg/kg/day, preferably about 0.1mg/kg/day when administered parenterally and about 50mg/kg/day when administered orally.

## Claims (Claims for the following Contracting State(s): AT, BE, CH, DE, FR, GB, IT, LI, LU, NL, SE)

1. A compound having the formula 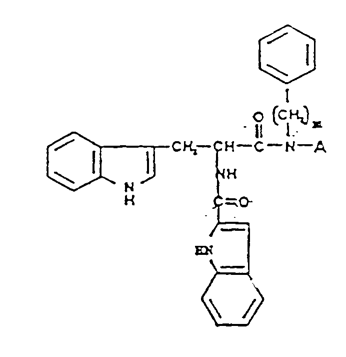 or pharmaceutically acceptable salt thereof, wherein m is 0, 1 or 2 inclusive;
A is either 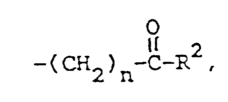 wherein n is an integer between 1 and 5, inclusive, and R² is hydroxy, an alkoxy group having 1-5, inclusive, carbon atoms, aralkoxy, aralkyl, amino, an alkyl group having 1-5, inclusive, carbon atoms, an alkylamino group having 1-5, inclusive, carbon atoms, a dialkylamino group with each alkyl group having 1-5, inclusive, carbon atoms, a cycloalkylamino group wherein the ring has 4-6, inclusive, carbon atoms, or morpholino; or A is an alkyl group having 1-5, inclusive, carbon atoms, a hydroxalkyl group having 1-5, inclusive, carbon atoms, an alkoxyalkyl group group having 2-8, inclusive, carbon atoms, an aralyoxyalkyl having 8-14, inclusive, carbon atoms, an aryl group having 6-14, inclusive, carbon atoms, an aralkyl group having 6-14, inclusive, carbon atoms, or a cycloalkyl group having 3-12, inclusive, carbon atoms.

2. The compound of claim 1, wherein the tryptophan residue is of the the L-configuration, m is 2, and A is 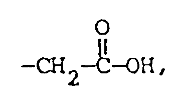 said compound having the name α-N-(2-indolylcarbonyl)-L-tryptyl-N'-phenylethylglycine; or a pharmaceutically acceptable salt thereof.

3. The compound of claim 1, wherein the tryptophan residue is of the L-configuration, m is 2, and A is 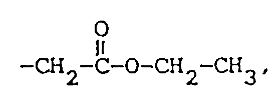 said compound having the name α-N-(2-indolylcarbonyl)-L-tryptyl-N'-phenylethylglycineethylester; or a pharmaceutically acceptable salt thereof.

4. The compound of claim 1, wherein m is 2,

5. The compound of claim 1, wherein A is 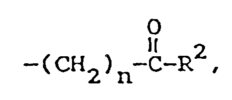 wherein R² is hydroxy or an alkoxy group having 1-5, inclusive, carbon atoms.

6. The compound of claim 1, wherein m is 1.

7. A pharmaceutical composition comprising a therapeutically effective amount of a compound according to any preceding claim together with a pharmaceutically acceptable carrier or diluent.

8. A compound as defined in any of Claims 1 to 6 for use in therapy or to suppress appetite.

9. A compound as defined in any of Claims 1 to 6 for use in the treatment of disorders, such as gastric, peptic or duodenal ulcers or acute pancreatitis, associated with excessive cholecystokinin.

10. Use of a compound as defined in any of Claims 1 to 6 for the manufacture of a medicament for use in the treatment of disorders, such as gastric, peptic or duodenal ulcers or acute pancreatitis, associated with excessive cholecystokinin, or for the suppression of appetite.

11. A method of synthesizing a compound as defined in Claim 1, comprising the step of condensing a compound of formula (5) 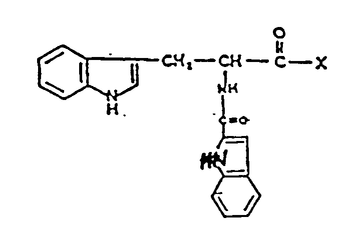 where X represents a hydroxyl or carboxylic acid activating group, with a secondary amino compound of formula (6) 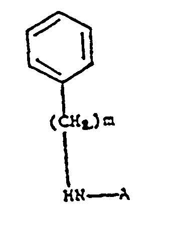 where m and A are defined as in Claim 1.

## Claims (Claims for the following Contracting State(s): ES, GR)

1. A method of producing a pharmaceutical composition comprises mixing a compound having the formula: 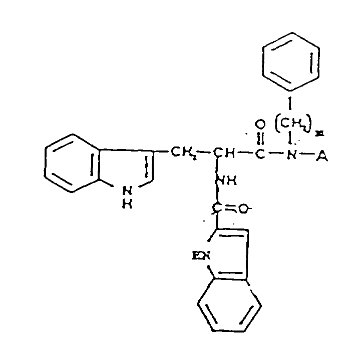 or pharmaceutically acceptable salt thereof, wherein m is 0, 1 or 2 inclusive;
A is either 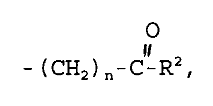 wherein n is an integer between 1 and 5, inclusive, and R² is hydroxy, an alkoxy group having 1-5, inclusive, carbon atoms, aralkoxy, aralkyl, amino, an alkyl group having 1-5, inclusive, carbon atoms, an alkylamino group having 1-5, inclusive, carbon atoms, a dialkylamino group with each alkyl group having 1-5, inclusive, carbon atoms, a cycloalkylamino group wherein the ring has 4-6, inclusive, carbon atoms, or morpholino; or A is an alkyl group having 1-5, inclusive, carbon atoms, a hydroxalkyl group having 1-5 inclusive, carbon atoms, an alkoxyalkyl group having 2-8, inclusive, carbon atoms, an aralkoxyalkyl having 8-14, inclusive, carbon atoms, an aryl group having 6-14, inclusive, carbon atoms, an aralkyl group having 6-14, inclusive, carbon atoms, or a cycloalkyl group having 3-12, inclusive, carbon atoms, with a pharmaceutically acceptable carrier or diluent.

2. The method of Claim 1, wherein the tryptophan residue is of the L-configuration, m is 2,
and A is 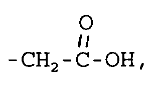 said compound having the name α-N-(2-indolylcarbonyl)-L-tryptyl-N'-phenylethylglycine; or a pharmaceutically acceptable salt thereof.

3. The method of Claim 1, wherein the tryptophan residue is of the L-configuration, m is 2,
and A is 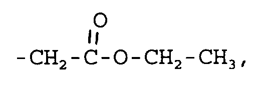 said compound having the name α-N-(2-indolylcarbonyl)-L-tryptypl-N'-phenylethylglycineethylester; or a pharmaceutically acceptable salt thereof.

4. The method of Claim 1, wherein m is 2.

5. The method of Claim 1, wherein A is 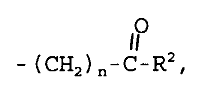 wherein R² is hydroxy or an alkoxy group having 1-5, inclusive, carbon atoms.

6. The method of Claim 1, wherein m is 1.

7. Use of a composition produced by the method of any of Claims 1 to 6 for the manufacture of a medicament for use in the treatment of disorders, such as gastric, peptic or duodenal ulcers or acute pancreatitis, associated with excessive cholecystokinin, or for the suppression of appetite.

8. A method of synthesizing a compound of the formula as defined in Claim 1, comprising the step of condensing a compound of formula (5) 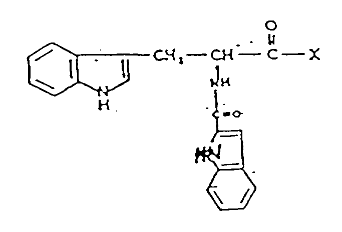 where X represents a hydroxyl or carboxylic acid activating group, with a secondary amino compound of formula (6) 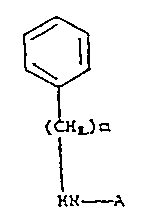 where m and A are defined as in Claim 1.

## Patentansprüche (Patentansprüche für folgende(n) Vertragsstaat(en): AT, BE, CH, DE, FR, GB, IT, LI, LU, NL, SE)

1. Verbindung der Formel 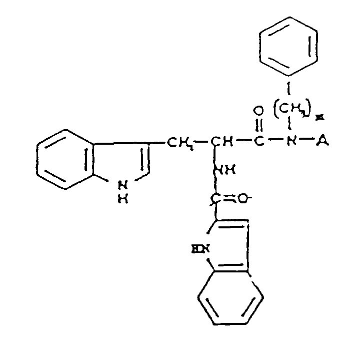 oder ein pharmazeutisch vertretbares Salz davon,
worin m für 0, 1 oder 2 steht,
A entweder 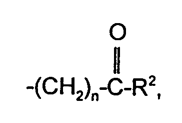 wobei n eine ganze Zahl zwischen 1
und 5 bedeutet, und R² Hydroxy, eine Alkoxygruppe mit 1 bis 5 Kohlenstoffatomen, Aralkoxy, Aralkyl, Amino, eine Alkylgruppe mit 1 bis 5 Kohlenstoffatomen, eine Alkylaminogruppe mit 1 bis 5 Kohlenstoffatomen, eine Dialkylaminogruppe, wobei jede Alkylgruppe 1 bis 5 Kohlenstoffatome aufweist, eine Cycloalkylaminogruppe, bei der der Ring 4 bis 6 Kohlenstoffatome aufweist, oder Morpholino ist, oder A eine Alkylgruppe mit 1 bis 5 Kohlenstoffatomen, eine Hydroxyalkylgruppe mit 1 bis 5 Kohlenstoffatomen, eine Alkoxyalkylgruppe mit 2 bis 8 Kohlenstoffatomen, eine Aralkoryalkylgruppe mit 8 bis 14 Kohlenstoffatomen, eine Arylgruppe mit 6 bis 14 Kohlenstoffatomen, eine Aralkylgruppe mit 6 bis 14 Kohlenstoffatomen oder eine Cycloalkylgruppe mit 3 bis 12 Kohlenstoffatomen ist.

2. Verbindung nach Anspruch 1, bei der der Tryptophanrest die L-Konfiguration aufweist, m 2 ist und A 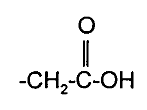 ist, wobei es sich bei der Verbindung um α-N-(2-Indolylcarbonyl)-L-tryptyl-N'-phenylethylglycin handelt, oder ein pharmazeutisch vertretbares Salz davon.

3. Verbindung nach Anspruch 1, bei der der Tryptophanrest die L-Konfiguration aufweist, m 2 ist und A 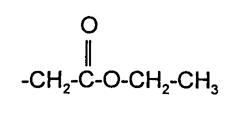 ist, wobei es sich bei der Verbindung um α-N-(2-Indolylcarbonyl)-L-tryptyl-N'-phenylethylglycinethylester handelt, oder ein pharmazeutisch vertretbares Salz davon.

4. Verbindung nach Anspruch 1, bei der m 2 ist.

5. Verbindung nach Anspruch 1, bei der A 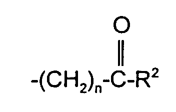 ist, wobei R² Hydroxy oder eine Alkoxygruppe mit 1 bis 5 Kohlenstoffatomen ist.

6. Verbindung nach Anspruch 1, bei der m 1 ist.

7. Pharmazeutische Zusammensetzung, die eine therapeutisch wirksame Menge einer Verbindung nach einem der vorstehenden Ansprüche zusammen mit einem pharmazeutisch vertretbaren Träger- oder Verdünnungsmittel umfaßt.

8. Verbindung nach einem der Ansprüche 1 bis 6 zum Einsatz für therapeutische Zwecke oder als Appetitszügler.

9. Verbindung nach einem der Ansprüche 1 bis 6 zum Einsatz bei der Behandlung von Erkrankungen, wie z.B. Magen-, peptisches oder Zwölffingerdanngeschwür oder akute Pankreatitis, die mit einem Cholezystokininüberschuß einhergehen.

10. Verwendung einer Verbindung nach einem der Ansprüche 1 bis 6 für die Herstellung eines Arzneimittels zum Einsatz bei der Behandlung von Erkrankungen, wie z.B. Magen-, peptisches oder Zwölffingerdarmgeschwür oder akute Pankreatitis, die mit einem Cholezystokininüberschuß einhergehen, oder als Appetitszügler.

11. Verfahren zur synthetischen Herstellung einer Verbindung nach Anspruch 1, das als Schritt die Kondensation einer Verbindung der Formel (5) 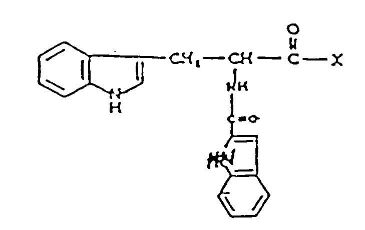 in der X für eine Hydroxyl- oder Carbonsäure aktivierende Gruppe steht, mit einer sekundären Aminoverbindung der Formel (6) 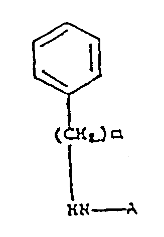 in der m und A der Definition in Anspruch 1 entsprechen, umfaßt.

## Patentansprüche (Patentansprüche für folgende(n) Vertragsstaat(en): ES, GR)

1. Verfahren zur Herstellung einer pharmazeutischen Zusammensetzung, welches das Mischen einer Verbindung der Formel 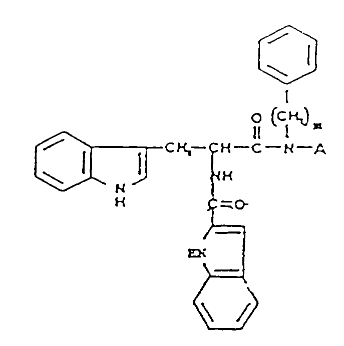 oder eines pharmazeutisch vertretbaren Salzes davon,
worin m für 0, 1 oder 2 steht,
A entweder 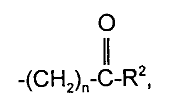 wobei n für eine ganze Zahl zwischen 1
und 5 steht, und R² Hydroxy, eine Alkoxygruppe mit 1 bis 5 Kohlenstoffatomen, Aralkoxy, Aralkyl, Amino, eine Alkylgruppe mit 1 bis 5 Kohlenstoffatomen, eine Alkylaminogruppe mit 1 bis 5 Kohlenstoffatomen, eine Dialkylaminogruppe, wobei jede Alkylgruppe 1 bis 5 Kohlenstoffatome aufweist, eine Cycloalkylaminogruppe, bei der der Ring 4 bis 6 Kohlenstoffatome aufweist, oder Morpholino ist, oder A eine Alkylgruppe mit 1 bis 5 Kohlenstoffatomen, eine Hydroxyalkylgruppe mit 1 bis 5 Kohlenstoffatomen, eine Alkoxyalkylgruppe mit 2 bis 8 Kohlenstoffatomen, eine Aralkoxyalkylgruppe mit 8 bis 14 Kohlenstoffatomen, eine Arylgruppe mit 6 bis 14 Kohlenstoffatomen, eine Aralkylgruppe mit 6 bis 14 Kohlenstoffatomen oder eine Cycloalkylgruppe mit 3 bis 12 Kohlenstoffatomen ist, mit einem pharmazeutisch vertretbaren Träger- oder Verdünnungsmittel umfaßt.

2. Verfahren nach Anspruch 1, bei dem der Tryptophanrest die L-Konfiguration aufweist, m 2 ist und A 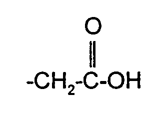 ist, wobei es sich bei der Verbindung um α-N-(2-Indolylcarbonyl)-L-tryptyl-N'-phenylethylglycin handelt, oder ein pharmazeutisch vertretbares Salz davon.

3. Verfahren nach Anspruch 1, bei dem der Tryptophanrest die L-Konfiguration aufweist, m 2 ist und A 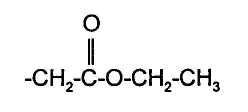 ist, wobei es sich bei der Verbindung um α-N-(2-Indolylcarbonyl)-L-tryptyl-N'-phenylethylglycinethylester handelt, oder ein pharmazeutisch vertretbares Salz davon.

4. Verfahren nach Anspruch 1, bei dem m 2 ist.

5. Verfahren nach Anspruch 1, bei dem A 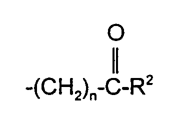 ist, wobei R² Hydroxy oder eine Alkoxygruppe mit 1 bis 5 Kohlenstoffatomen ist.

6. Verfahren nach Anspruch 1, bei dem m 1 ist.

7. Verwendung einer Zusammensetzung, die nach dem Verfahren nach einem der Ansprüche 1 bis 6 hergestellt wurde, für die Herstellung eines Arzneimittels zur Behandlung von Erkrankungen, wie z.B. Magen-, peptisches oder Zwölffingerdarmgeschwür oder akute Pankreatitis, die mit einem Cholezystokininüberschuß einhergehen, oder zum Einsatz als Appetitszügler.

8. Verfahren zur synthetischen Herstellung einer Verbindung nach Anspruch 1, das als Schritt die Kondensation einer Verbindung der Formel (5) 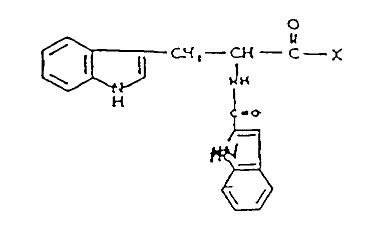 in der X für eine Hydroxyl- oder Carbonsäure aktivierende Gruppe steht, mit einer sekundären Aminoverbindung der Formel (6) 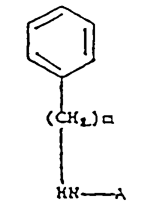 in der m und A der Definition in Anspruch 1 entsprechen, umfaßt.

## Revendications (Revendications pour l'(les) Etat(s) contractant(s) suivant(s): AT, BE, CH, DE, FR, GB, IT, LI, LU, NL, SE)

1. Composé présentant la formule : 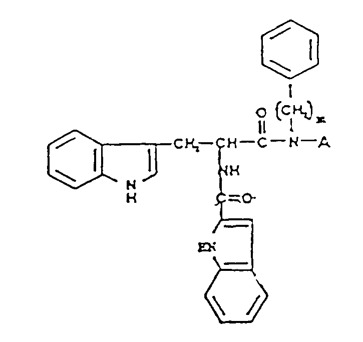 ou sel pharmaceutiquement acceptable de celui-ci, dans lequel m est 0, 1 ou 2 inclus ;
A est 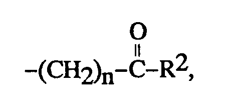 où n est un entier compris entre 1 et 5 inclus et R² est hydroxyle, un groupe alcoxy ayant 1 à 5 atomes de carbone inclus, aralcoxy, aralkyle, amino, un groupe alkyle ayant 1 à 5 atomes de carbone inclus, un groupe alkylamino ayant 1 à 5 atomes de carbone inclus, un groupe dialkylamino dans lequel chaque groupe alkyle a 1 à 5 atomes de carbone inclus, un groupe cycloalkylamino dans lequel le cycle a 4 à 6 atomes de carbone inclus, ou morpholino, ou bien A est un groupe alkyle ayant 1 à 5 atomes de carbone inclus, un groupe hydroxyalkyle ayant 1 à 5 atomes de carbone inclus, un groupe alcoxyalkyle ayant 2 à 8 atomes de carbone inclus, un groupe aralcoxyalkyle ayant 8 à 14 atomes de carbone inclus, un groupe aryle ayant 6 à 14 atomes de carbone inclus, un groupe aralkyle ayant 6 à 14 atomes de carbone inclus ou un groupe cycloalkyle ayant 3 à 12 atomes de carbone inclus.

2. Composé selon la revendication 1, dans lequel le résidu tryptophane est de configuration L, m est égal à 2 et A est 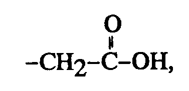 ledit composé ayant l'appellation α-N-(2-indolylcarbonyl)-L-tryptyl-N'-phényléthylglycine, ou sel pharmaceutiquement acceptable de celui-ci.

3. Composé selon la revendication 1, dans lequel le résidu tryptophane est de configuration L, m est égal à 2 et A est 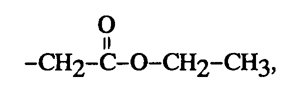 ledit composé ayant l'appellation éthylester de α-N-(2-indolylcarbonyl)-L-tryptyl-N'-phényléthylglycine, ou sel pharmaceutiquement acceptable de celui-ci.

4. Composé selon la revendication 1, dans lequel m est égal à 2.

5. Composé selon la revendication 1, dans lequel A est 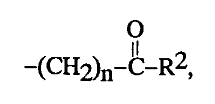 où R² est hydroxyle ou un groupe alcoxy ayant 1 à 5 atomes de carbone inclus.

6. Composé selon la revendication 1, dans lequel m est égal à 1.

7. Composition pharmaceutique comprenant une quantité thérapeutiquement efficace d'un composé selon l'une quelconque des revendications précédentes ainsi qu'un véhicule ou diluant pharmaceutiquement acceptable.

8. Composé selon l'une quelconque des revendications 1 à 6 destiné à être utilisé en thérapie ou pour supprimer l'appétit.

9. Composé selon l'une quelconque des revendications 1 à 6 destiné à être utilisé dans le traitement de troubles, tels que les ulcères gastriques, gastro-duodénaux ou duodénaux ou la pancréatite aiguë, associés à un exces de cholécystokinine.

10. Utilisation d'un composé selon l'une quelconque des revendications 1 à 6 pour la préparation d'un médicament destiné à être utilisé dans le traitement de troubles, tels que les ulcères gastriques, gastro-duodénaux ou duodénaux ou la pancréatite aiguë, associés à un excès de cholécystokinine, ou pour la suppression de l'appétit.

11. Procédé de synthèse d'un composé selon la revendication 1, comprenant l'étape de condensation d'un composé de formule (5) : 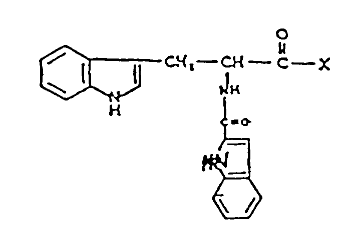 dans laquelle X représente un groupe activant les hydroxyles ou les acides carboxyliques, avec un composé amino secondaire de formule (6) : 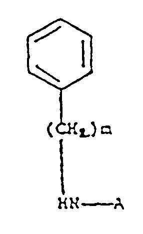 dans laquelle m et A sont définis comme dans la revendication 1.

## Revendications (Revendications pour l'(les) Etat(s) contractant(s) suivant(s): ES, GR)

1. Procédé de production d'une composition pharmaceutique qui comprend le mélange d'un composé de formule : 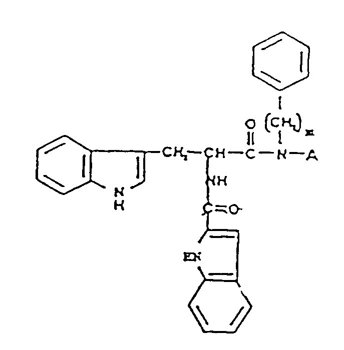 ou d'un sel pharmaceutiquement acceptable de celui-ci, dans lequel m est égal 0, 1 ou 2 inclus;
A est 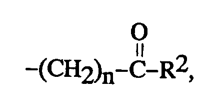 où n est un entier compris entre 1 et 5 inclus et R² est hydroxyle, un groupe alcoxy ayant 1 à 5 atomes de carbone inclus, aralcoxy, aralkyle, amino, un groupe alkyle ayant 1 à 5 atomes de carbone inclus, un groupe alkylamino ayant 1 à 5 atomes de carbone inclus, un groupe dialkylamino dans lequel chaque groupe alkyle a 1 à 5 atomes de carbone inclus, un groupe cycloalkylamino dans lequel le cycle a 4 à 6 atomes de carbone inclus, ou morpholino, ou bien A est un groupe alkyle ayant 1 à 5 atomes de carbone inclus, un groupe hydroxyalkyle ayant 1 à 5 atomes de carbone inclus, un groupe alcoxyalkyle ayant 2 à 8 atomes de carbone inclus, un groupe aralcoxyalkyle ayant 8 à 14 atomes de carbone inclus, un groupe aryle ayant 6 à 14 atomes de carbone inclus, un groupe aralkyle ayant 6 à 14 atomes de carbone inclus ou un groupe cycloalkyle ayant 3 à 12 atomes de carbone inclus, avec un véhicule ou diluant pharmaceutiquement acceptable.

2. Procédé selon la revendication 1, dans lequel le résidu tryptophane est de configuration L, m est égal à 2 et A est 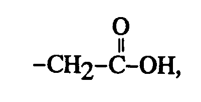 ledit composé ayant l'appellation α-N-(2-indolylcarbonyl)-L-tryptyl-N'-phényléthylglycine, ou sel pharmaceutiquement acceptable de celui-ci.

3. Procédé selon la revendication 1, dans lequel le résidu tryptophane est de configuration L, m est égal à 2 et A est 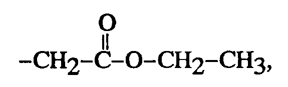 ledit composé ayant la dénomination éthylester d'α-N-(2-indolylcarbonyl)-L-tryptyl-N'-phényléthylglycine, ou sel pharmaceutiquement acceptable de celui-ci.

4. Procédé selon la revendication 1, dans lequel m est égal à 2.

5. Procédé selon la revendication 1, dans lequel A est 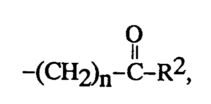 où R² est hydroxyle ou un groupe alcoxy ayant 1 à 5 atomes de carbone inclus.

6. Procédé selon la revendication 1, dans lequel m est égal à 1.

7. Utilisation d'une composition produite par le procédé selon l'une quelconque des revendications 1 à 6 pour la préparation d'un médicament destiné à être utilisé dans le traitement de troubles, tels que les ulcères gastriques, gastro-duodénaux ou duodénaux ou la pancréatite aiguë, associés à un excès de cholécystokinine, ou pour la suppression de l'appétit.

8. Procédé de synthèse d'un composé de formule telle que définie dans la revendication 1, comprenant l'étape de condensation d'un composé de formule (5) : 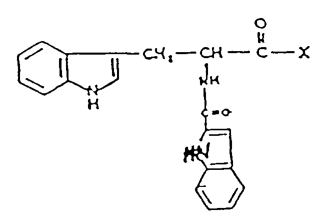 dans laquelle X représente un groupe activant les hydroxyles ou les acides carboxyliques, avec un groupe amino secondaire de formule (6) : 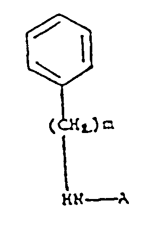 dans laquelle m et A sont définis comme dans la revendication 1.
